# EUROPEAN PATENT SPECIFICATION

(11) **EP 3 169 443 B1**
(45) Date of publication and mention of the grant of the patent: **14.09.2022**
(21) Application number: 15751132.0
(22) Date of filing: 15.07.2015
(51) Int. Cl.: B05B 7/04, B65D 83/30, B05B 1/16, B05B 1/28, B05B 7/24, B65D 83/36, B65D 83/44, B65D 83/14, A61B 90/00, A61M 11/02, A61M 19/00, A61M 35/00

(54) **NEBULIZER AND SPACER FOR THE TOPICAL APPLICATION OF A LIQUID AND/OR SOLID TO A SURFACE**
VERNEBLER UND ABSTANDSHALTER FÜR DIE TOPISCHE ANWENDUNG EINER FLÜSSIGKEIT UND/ODER EINES FESTSTOFFES AUF EINE OBERFLÄCHE
NÉBULISEUR ET ÉLÉMENT D'ESPACEMENT POUR L'APPLICATION TOPIQUE D'UN LIQUIDE ET/OU D'UN SOLIDE SUR UNE SURFACE

(30) Priority: 15.07.2014 NL 2013186
(43) Date of publication of application: 24.05.2017
(73) Proprietor: Dutch Renewable Energy B.V., 1398 CP Muiden (NL)
(72) Inventor: KLEVER, Diede Hendrik Paul, 1053 LD Amsterdam (NL); PELLIKAAN, Hubert Clemens, 3572 CA Utrecht (NL); VISSER, Rolf Lourens, 3621 ND Breukelen (NL)
(74) Representative: Nederlandsch Octrooibureau
(86) International application number: PCT/NL2015/050517
(87) International publication number: WO 2016/010428

(56) References cited:
- WO-A1-90/05580
- GB-A- 1 263 521
- GB-A- 1 555 771
- GB-A- 2 018 908
- GB-A- 2 410 707
- GB-A- 2 458 128
- JP-A- H0 824 729
- JP-A- H07 275 759
- JP-A- H08 299 866
- US-A1- 2013 214 060
- US-B1- 6 883 688

## Description

The invention relates to a nebulizer for the topical application of a liquid cooling medium. The invention also relates to the use of such a nebulizer for topical application of a liquid cooling medium to the skin of a person or an animal.

Various nebulizers, such as spray cans, are already known for many diverse applications. Such nebulizers comprise a container in which liquid is kept under pressure with a propellant gas or compressed air in order to expel the liquid as a fine-particle spray. To expel the liquid as a fine-particle spray, the known nebulizer has a spray nozzle with a specific bore. The size of the bore created in the nozzle is generally of determining significance for the dispersion of the spray as well as the liquid concentration (for example the number of milligrams per second) in the spray generated by the nozzle.

GB 1 263 521 describes a fluids dispensing valve which comprises a housing, a popper member reciprocably mounted in said housing, a flexible valve element having first and second sides, an annular valve seat means in said housing engageable by said first side of said flexible valve element, a throughway in the valve element and in alignment with the inside of the annulus of the seat, said popper member being engageable with said second side of said flexible valve element to seal the element to the annular seat and to seal the throughway, a spring tending to force said poppet member into sealing engagement with said flexible valve element, a first inlet passage leading to the first side of the valve element on the outside of the annulus ef the seat, a second inlet passage leading to the second side of the valve element and a third outlet passage leading from the inside of the annulus and actuator means for moving said poppet member away from said flexible valve element to allow said first and second passages to communicate with the third passage.

GB 2 018 908 describes an improved valve for the admixture of a gas with a fluid stored under pressure in a container, comprising a hollow body associated with a drawing tube for the fluid transport or conveyance and provided with at least one through hole forthe gas transport or conveyance, as well as a shutter element movable in said body against a spring and provided with a hollow stem having at least one opening that can be shut off by a sealing gasket, wherein said body has therein a nozzle which is hydraulically connected with the drawing tube and cooperates with a venturi effect conduit which, together with said nozzle, defines an annular space communicating with said through hole for the gas transport or conveyance.

GB 2 410 707 describes a wound treatment device comprising a housing having a first opening and a second opening, wherein the device further comprises an adhesive-coated flange around the first opening for attachment of the device around a wound to be treated, and wherein the second opening is adapted for attachment to an aerosol container.

By decreasing the bore size of the nozzle it is possible to create a nebulizer that is designed for generating a spray comprising a low liquid concentration and/or that is designed for the topical, i.e., highly local application of a liquid and/or solid substance.

EP 1 867 295 A1 describes a device for cold treating a tissue and a method for cold treating a tissue.

The present invention provides an improved nebulizer as defined in the claims.

The nebulizer of the present invention has the advantage that, due to the formation of a colloidal mixture comprising a dispersed phase consisting of the liquid cooling medium and a continuous phase consisting of the propellant gas before delivering the colloidal mixture to the nozzle, the size of the bore of the nozzle does not necessarily have to be adjusted in order to generate a spray with a reduced concentration of liquid and/or to generate a spray that can be applied topically, i.e., very locally, to a surface. The present invention therefore provides a nebulizer that is designed for the highly directed treatment of surfaces.

In an embodiment according to the present invention the contents of the container and, optionally, the actuatable valve attached to the container are held under a pressure greater than atmospheric pressure. Preferably, in the closed state the pressures in the container and in the actuatable valve attached to the container are the same. It is to be noted that if the valve is in the opened state, the pressure in the valve will be lower than the pressure in the container. More preferably the pressure referred to above is greater than 2.0 bar, preferably greater than 3.0 bar, preferably approximately 4.0 bar, preferably approximately 5.0 bar, preferably approximately 12 bar, preferably approximately 60 bar.

The colloidal mixture such as that formed in the mixing chamber comprises a dispersed phase of liquid cooling medium and a continuous phase of propellant gas. It has been found that a fine spray can be produced which has the properties of a directed liquid stream. The colloidal mixture such as that formed in the mixing chamber can be of various types, but preferably comprises an aerosol.

In order to guarantee the delivery of a colloidal mixture comprising a dispersed phase consisting of a liquid cooling medium and a continuous phase consisting of a propellant gas, the first and/or second inlet attached to the mixing chamber is preferably provided with inlet blocking means, such as a shut-off valve that is opened when the device is brought into the proper orientation for use. Proper orientation is defined here as the position in which the first and second inlets are positioned such that they supply the liquid cooling medium and the propellant gas via the inlets intended for them in order to form the desired colloidal mixture. Such a blocking means preferably comprises a ball, movable under the influence of gravity, which closes the first and/or second inlet when the nebulizer is in an inactive orientation. Such an inactive orientation comprises for example the orientation of the nebulizer wherein the nozzle is directed in the opposite direction to the direction of the force of gravity. As soon as the nebulizer is rotated into an active orientation (wherein the nozzle is facing in a direction substantially identical to the direction of the force of gravity), the first and/or second inlet that is closed in the inactive orientation is opened and makes the use of the nebulizer possible.

The stream generated by the nozzle of the nebulizer can have the shape of a cone with a top angle of the cone that is smaller than 30°, preferably smaller than 20° and preferably smaller than 10°, preferably smaller than 5°. The nebulizer according to the present invention thus provides a stream that can be directed against a surface to be treated. The top angle of the cone may also optionally be greater than 30°.

To further prevent unintentional discharge of the nebulizer, in one embodiment the nebulizer of the present invention comprises a blocking element. Such a blocking element is designed for blocking the valve in at least one position. A blocking element can for example consist of a lever or slide mechanism, in which the lever or the slide mechanism is placed in a blocking position in the space formed between the bottom edge of the nozzle and the top edge of the container. In this way the lever or the slide mechanism prevents the nozzle from being pressed in to open the valve and activate the nebulizer. The nebulizer can be unlocked by removing the lever or the slide mechanism from the space formed between the bottom edge of the nozzle and the top edge of the container. In the unlocked position the nozzle can be pressed in to open the valve and activate the nebulizer.

The container of the nebulizer is filled with a liquid cooling medium which cools the skin by evaporation when applied to the skin. In the case of various skin problems, the local withdrawal of heat from the skin can have a remediating or at least an alleviating effect. Examples of skin problems wherein the local cooling of the skin has a remediating or at least an alleviating effect comprise for example (topical) burns, pustules (for example caused by acne), itching (for example nettle rash) and skin irritation due to an insect bite. If very low temperatures can be achieved during the topical application of a cooling medium to the skin, it is also possible to treat skin conditions such as warts. The treatment of skin conditions such as warts takes place at temperatures below the freezing point.

The above-described treatment of skin conditions by the application of a cooling medium to the skin of a person or animal is better known as cryotherapy. In cryotherapy use is frequently made of liquid nitrogen (boiling point: -196°C) applied with a cotton swab or a nozzle specially designed for the application of nitrogen. Under the marked change in temperature and/or ambient pressure the nitrogen evaporates on contact with the skin, with the result that the skin cools markedly. In addition to liquid nitrogen, use can also be made of a cooling medium with a considerably higher boiling point, such as dimethyl ether, propane and mixtures thereof (boiling point: -57°C). Mixtures with such boiling points are commercially available.

The ability to apply a cooling medium in a directed manner prevents the surrounding skin, which does not need to be treated, from coming into contact with the cooling medium. Especially when temperatures below the freezing point are used, it is desirable to prevent contact of the surrounding skin with the cooling medium in order to increase the user's comfort. Since freezing of the cooling medium starts as soon as it leaves the nozzle, if the bore of the nozzle is too small, the nozzle will become blocked by frozen cooling medium. By creating a nebulizer in which the valve comprises a mixing chamber that is designed for the immediate formation of a colloidal mixture, cooling medium can be applied to the skin to be treated in a directed manner without requiring a nozzle bore size that can result in clogging of the nozzle.

The valve of the nebulizer according to the present invention is preferably designed for the uninterrupted, i.e., continuous, application of cooling medium. Through the uninterrupted, continuous, delivery of the cooling medium a lower temperature can be achieved than is usual at this time. With a metered dose, i.e., a predetermined quantity, limited cooling of the skin is accomplished. This limited cooling generally achieves a temperature of down to -20°C. Through the uninterrupted delivery of the cooling medium a temperature of below -20°C can be achieved. Preferably the temperature achieved is below -30°C. Through the uninterrupted delivery of cooling medium a temperature can be achieved which corresponds to the boiling point of the propellant gas, but it is also possible to reach temperatures that are below the boiling point of the propellant gas, for example by further expansion of the propellant gas. For example a temperature of approximately -50°C can be achieved with 1,1,1,2-tetrafluoroethane (R134a, boiling point: -26.3°C) as propellant gas.

In one variant embodiment the cooling medium comprises at least one active compound comprising an antiviral agent, terpene and/or essential oil. The advantage of a cooling medium comprising at least one active compound is that in addition to cooling the skin of the user, the skin is also treated with an active compound that has an additional remediating effect.

Preferably the terpene and the essential oil are chosen from camphor, menthol, thymol, thyme oil, eucalyptus, eucalyptus citriodora, turpentine, pine oil, Melaleuca alternifolia, menthone, menthyl salicylate, musk oil, bixa orellana, borneol, curcuma oil, peppermint oil, clove oil, fennel oil, basil oil, patchouli oil, alpha-pinene, terpineol, oregano oil, carvacrol and combinations thereof.

The antiviral agents preferably comprise replication inhibitors, such as cidofovir, acyclovir, pencyclovir and the like. However, other antiviral agents may also be considered.

The cooling medium of the device of the present invention preferably contains a propellant gas which comprises a mixture of hydrocarbons. In one embodiment the propellant gas comprises a cooling agent, in particular a cooling agent with a boiling point below -10°C. Examples of suitable cooling agents of this type comprise propane, n-butane, 1,1,1,2-tetrafluoroethane, dimethyl ether, dimethyl ether/propane mixtures and combinations thereof. As was stated in the preceding, the contents of the container are preferably kept under a pressure greater than atmospheric pressure in order to achieve rapid evaporation of the propellant gas upon application of the cooling medium to the skin.

It has been found that if a cooling medium is used to cool the skin to be treated, the temperature to be reached depends on the distance between the nozzle of the nebulizer and the surface to be treated. Therefore the invention provides a nebulizer comprising a spacer which is designed for adjustment of the distance between the nozzle of the nebulizer and the surface to be treated. By positioning the nozzle at a greater distance from the surface to be treated, a lower temperature can be achieved than if the surface to be treated is located at a shorter distance from the nozzle. For this purpose the spacer preferably comprises a displacing element for adjustment of the distance between the surface to be treated and the nozzle, i.e., varying the length of the spacer in the lengthwise direction.

In a preferred embodiment, the size of the opening provided in the cover means is variable.

The spacer of the present invention has the advantage that as a result of the ability to vary the size of the opening provided in the cover means, the liquid cooling medium to be applied to a surface can be regulated. In consequence it is possible by means of the spacer of the present invention to accurately determine the surface to be treated. In this way it is possible to prevent the surrounding surface, which is not to be treated, from coming into contact with the liquid cooling medium. In particular, when a cooling medium such as that described above is used, wherein temperatures below the freezing point are achieved, it is desirable to prevent contact of the surrounding skin with the cooling medium in order to enhance comfort for the user.

In one embodiment the valve and/or the nozzle of the nebulizer that can be connected to the spacer can be coupled with an actuating element, which actuating element is integrated in the spacer. In one embodiment variant the spacer comprises an actuating element that extends outside of the spacer. Such an actuating element can, for example, be made in the form of a control button or lever. The actuation of a stream or spray can be accomplished by pressing the control button or moving the lever. To permit easy operation of the spacer and the nebulizer that can be coupled to it, the actuating element is preferably integrated in the spacer. In such a variant embodiment wherein the actuating element is integrated in the spacer, it is possible to actuate the generation of a stream or medium by pressing the container of the nebulizer toward the spacer. The application of a stream or spray of liquid cooling medium to the skin in such a manner prevents the initial position of the spacer set by the user and the opening located in the cover means relative to the surface to be treated, such as the skin, from being changed unintentionally at the time of actuation of the nebulizer. In addition, upon removal of the nebulizer, whether deliberately or unintentionally, the inlet of the stream or spray will be blocked. Such an integration of the actuating element in the spacer therefore prevents the unintentional application of liquid cooling medium to surrounding surfaces such as the skin or sensitive organs such as the eye of the user, to the benefit of safety for the user and the accuracy of the process.

As was already mentioned above, the spacer of the present invention is designed for fixing the minimal distance between the nozzle of the container and the surface to be treated or the opening provided in the cover means. In one embodiment the distance between the nozzle of the container and the surface to be treated or the opening provided in the cover means is approximately 0.5 cm to approximately 8.0 cm. It should be noted that to be able to optimally apply a cooling medium to the skin of a person or animal, the distance is preferably approximately 0.5 cm to approximately 7.0 cm, approximately 1.0 cm to approximately 4.0, approximately 1.5 cm to approximately 3.0 cm or approximately 2.0 cm.

The spacer of the present invention may take various shapes. Preferably the shape of the spacer provides that the stream or spray to be generated will have clear access to the opening formed in the cover means connected with the spacer. The shape of the spacer can therefore be selected from a cylinder, truncated cone, hyperboloid of one sheet, elliptical paraboloid and variants thereof. The spacer can have a completely closed shape, which prevents the user from accidentally touching the stream or spray for example with the fingers during the application of the stream or spray onto a surface to be treated. However, the spacer can also be provided with openings or a very open shape to facilitate actuation of the valve by the user and/or to promote evaporation of for example the propellant gas or cooling medium or to observe the stream more clearly.

Like the spacer, the cover means connected to the spacer can assume various shapes. Preferably the size of the cover means is greater than the maximal bore size that the stream or spray can assume upon reaching the opening introduced in the cover means. Preferably the cover means is integrated in the form of the spacer.

In one variant embodiment according to the present invention the cover means comprises at least two screen parts movable relative to one another into at least two different positions. Such screen parts are provided for enclosing openings with different dimensions when the screen parts are brought into different positions. The screen parts of the present invention can have different shapes. In one variant embodiment the screen parts overlap one another in all positions. Preferably the screen parts form a diaphragm. A diaphragm of this type provides the possibility for the user to accurately establish the area to be treated.

In one variant embodiment the cover means comprises at least one screen element adjustable strip provided with openings of different dimensions. Preferably the adjustable strip can be moved by guide means belonging to the cover means and/or spacer. Such a screen element in the form of an adjustable strip provides the possibility to create openings with different (predetermined) shapes. In addition, the use of an adjustable strip with openings of different dimensions decreases the likelihood of jamming of the different screen parts as a result of repeated use of the device. Preferably the adjustable strip comprises an adjusting knob for manually moving the adjustable strip.

The opening provided in the cover means according to the present invention, which is bisected by the center axis of the stream, is preferably larger than the largest opening provided in the adjustable strip.

In addition it is possible to arrange the adjustable strip in such a manner that in at least one position the adjustable strip is not provided with an opening through which it is possible to close the opening provided in the cover means. Such a position of the adjustable strip therefore prevents unintentional contact with the stream or spray to be generated by the nebulizer if the nebulizer is accidentally activated.

In an embodiment wherein the actuating element is integrated in the spacer, the spacer is preferably connected to the blocking element, such as a delimiting ring which is placed around the container and provided with a groove extending radially around the center axis, in which the spacer can be rotated radially around the center axis of the nebulizer. Axial movement of the spacer is blocked by the delimiting ring, thus preventing unintentional actuation of the nebulizer. In at least one position the blocking element is provided with an unlocking part, such as a groove extending in the axial direction connected with the groove extending in the radial direction, which makes possible axial movement of the spacer and actuation of the device.

Preferably the container of the nebulizer is provided with a cooling agent for the treatment of a wart located on the skin of a person or animal wherein the opening provided in the cover means is sized so as to only expose the wart. The treatment wherein the opening of the device is sized so as to only expose the wart provides the possibility of only treating the wart without the surrounding skin simultaneously coming into contact with the cooling medium, which increases the comfort for the user.

The present invention further relates to the use of a nebulizer for topical application of a liquid coolig medium to the skin of a person or animal, as defined in the claims.

In an embodiment of the use according to the present invention, after placing the cover means of the spacer on the skin, the one opening is brought into overlap with the center axis of the nebulizer, wherein the size of the opening corresponds to the size of the area to be cooled.

During topical applicatio the cooling medium can also be supplied in predetermined quantities. However, it is preferred for the cooling medium to be delivered without interruption during topical application. Through the uninterrupted, continuous delivery of the cooling medium a lower temperature can be achieved than is usual at this time. Limited cooling of the skin is achieved with a metered dose, such as a predetermined quantity. This limited cooling generally achieves a temperature of down to -20°C. By uninterrupted delivery of the cooling medium a temperature of below -20°C can be achieved. Preferably the temperature achieved is below -30°C. Through the uninterrupted delivery of cooling medium a temperature can be achieved which corresponds to the boiling point of the propellant gas, or even a temperature below the boiling point of the propellant gas.

In an embodiment of the use according to the present invention the delivery of the cooling medium is released during pressing of the valve and/or nozzle of the nebulizer.

The present invention furthermore provides a cooling medium for use in a nebulizer according to the present invention, comprising a colloidal mixture comprising a liquid cooling medium and a propellant gas and at least one active compound. Preferably the active compound comprises a substance that has antiviral activity. The cooling medium according to the present invention has the advantage that in addition to the remediating and/or alleviating effect from cooling the skin of the user by the propellant gas, the skin is also treated with an active compound that has a supplemental remediating effect. Thus an assembly of this type provides the possibility of allowing specific treatment for specific skin conditions. In an embodiment of the present invention the cooling medium comprises an active compound comprising an antiviral agent, terpene and/or essential oil.

As was stated previously, the antiviral agent can preferably comprise replication inhibitors such as cidofovir, acyclovir, pencyclovir and the like. However, other antiviral agents are also conceivable. If an active compound consisting of terpene and/or essential oils is used, they are preferably selected from camphor, menthol, thymol, thyme oil, eucalyptus, eucalyptus citriodora, turpentine, pine oil, Melaleuca alternifolia, menthone, menthyl salicylate, musk oil, bixa orellana, borneol, curcuma oil, peppermint oil, clove oil, fennel oil, basil oil, patchouli oil, alpha-pinene, terpineol, oregano oil, carvacrol and combinations thereof.

As was previously stated, the cooling medium may comprise a propellant gas which comprises a mixture of hydrocarbons. In one embodiment the propellant gas comprises a cooling agent, in particular, a cooling agent with a boiling point below -10°C. Examples of such suitable cooling agents comprise propane, n-butane, 1,1,1,2-tetrafluoroethane, dimethyl ether, dimethyl ether/propane mixture and combinations thereof. As was stated in the preceding, the contents of the container are preferably kept under a pressure greater than atmospheric pressure in order to achieve rapid evaporation of the propellant gas during the application of the cooling medium to the skin.

The nebulizer according to the present invention can be used for various applications. In order to further clarify the use of the nebulizer according to the present invention, some specific application information will be presented here. The nebulizer can be used for the application of cosmetics, such as eye shadow or foundation, or a therapeutically active substance to a body surface such as the skin.

The nebulizer according to the present invention is preferably used for the treatment of skin conditions, such as pigmented spots or moles,.

In the following, the present invention will be explained further based on the accompanying drawings, which show the following:
- Figure 1:: a schematic representation of the nebulizer according to the present invention;
- Figure 2:: a perspective view of an embodiment of the assembly according to the present invention;
- Figure 3:: a perspective view of a detail of an embodiment of part of the assembly according to the present invention;
- Figure 4:: a perspective view of a embodiment of the assembly according to the present invention;
- Figures 5a-b:: a rendering of a top view of an embodiment of the assembly according to the present invention;
- Figure 6:: a perspective view of a spacer of adjustable length; and
- Figure 7:: a schematic representation of the nebulizer according to the present invention with a controllable liquid inlet.

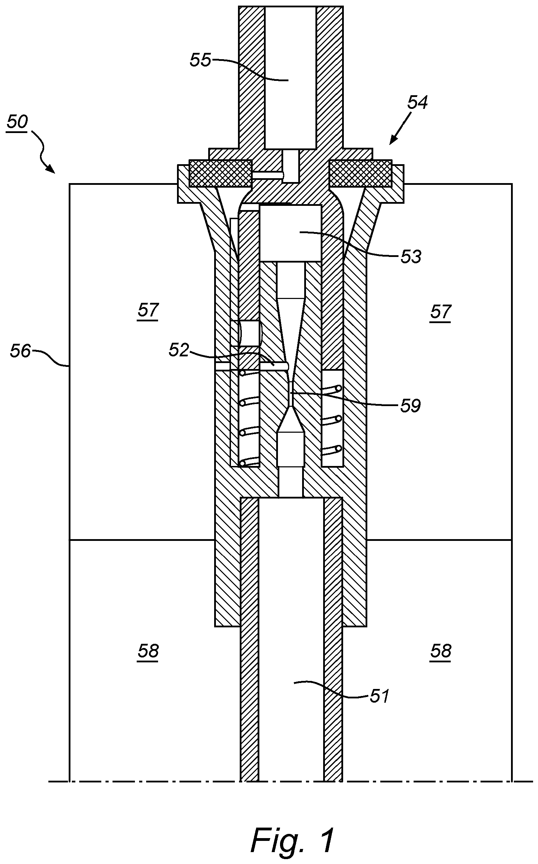

Figure 1 shows an embodiment of the nebulizer 50 according to the present invention. The nebulizer 50 comprises a first inlet 51, shown here in the form of a tube, and a second inlet 52. Both inlets 51, 52 are connected to a mixing chamber 53 which is incorporated in the valve 54. The valve 54 also comprises a nozzle 55 which is connected to the mixing chamber 53. The container 56 in which the valve 54 is placed comprises a propellant gas 57 (continuous phase) with a liquid 58 (dispersed phase). It is to be noted that the valve also comprises a Venturi 59 which is designed for drawing up liquid 58 when the valve is opened. The assembly as illustrated in figure 1 is most efficient in use when the nebulizer 50 is established with the nozzle 55 pointed downward (i.e., at least a position in which the nozzle 55 is at a lower level than the container 56 connected to the valve 54). In such use, upon activating the nebulizer, propellant gas 57 is delivered via the first inlet 51 to which the liquid is to be drawn up by the Venturi effect via second inlet 52. Since the mixing chamber 53 is located inside of the container 56, it should be noted that the pressure in the mixing chamber 53 should be largely equal to the pressure exerted on the liquid 58 and propellant gas 57 located in the container 56.

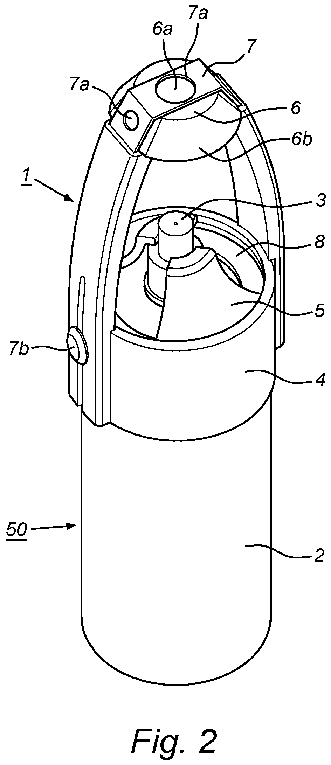

Figure 2 shows an embodiment of the assembly 1 according to the present invention in which the spacer 4 is connected to a nebulizer 50 having a container 2. The container 2 as illustrated in figure 2 refers to a container for a spray can, and is therefore usually cylindrical in shape. However, any arbitrary shape can be used. The container 2 preferably contains a cooling medium in the form of a liquid which is held under pressure. The nebulizer 50 also comprises a nozzle 3 connected by means of a valve (not shown here) to the container, which upon actuation of the nebulizer 50 produces a directed stream of cooling medium. The assembly 1 also comprises a spacer 4 which in figure 2 has a very open elliptical paraboloid shape. In addition the spacer has an integrated actuating element 5 which is connected to the tip 3. The actuating element 5 triggers the generation of a stream of cooling medium by pressing in the tip 3 (and the valve, not shown). In figure 2 the actuating element 5 is integrated in the spacer 4, which has the result that a stream of cooling medium can be generated either by pressing in the container 2 or by pressing in the spacer 4. The spacer 4 is connected to a cover means 6. The cover means 6 can assume the form of a cap, wherein the cover means also comprises an opening 6a (the outline of which is not visible in figure 2). Preferably the cover means 6 comprises an edge 6b directed toward the outside, which obstructs the application of cooling medium on the surrounding skin. The spacer 4 also comprises an adjustable strip 7 which is provided with openings 7a of different dimensions. Figure 2 illustrates two such openings 7a. The adjustable strip 7 can be adjusted by the user by moving the adjusting knob 7b. The spacer 4 is provided with guides (not illustrated), which guide the adjustable strip 7 during the moving of the adjustable strip 7 and the positioning of the desired opening 7a in front of the opening 6a of the cover means 6. The assembly 1 of figure 2 also comprises a delimiting ring 8 for blocking the spacer 4 in at least one position.

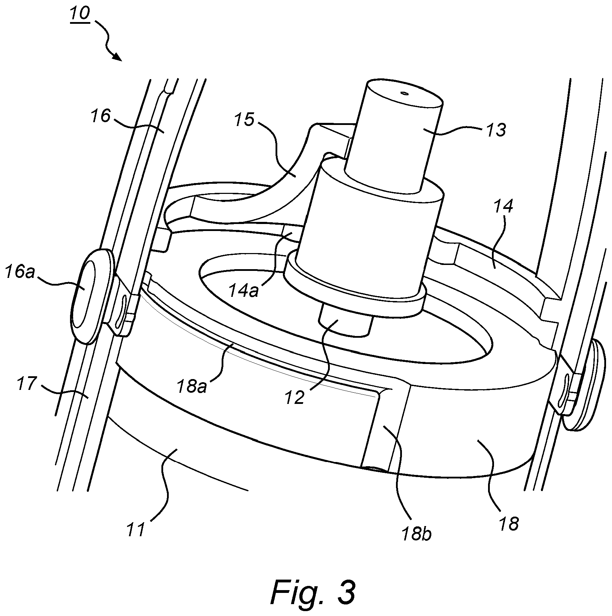

Figure 3 shows a detailed rendering of part of an embodiment of the assembly 10 according to the present invention. In figure 3 the nozzle 13 is connected to a valve 12 which is connected to the container 11, which container contains a cooling medium (not illustrated here). Figure 3 also shows a part of the spacer 14 which comprises an integrated actuating element 15. In addition, the assembly 10 of figure 3 comprises an adjustable strip 16 which has an adjusting knob 16a. The openings of different dimensions made in the adjustable strip 16 are not shown in figure 3. The adjustable strip 16 can be moved by guiding in the guides 17 placed in the spacer 14. The assembly 10 also comprises a delimiting ring 18 which comprises a groove 18a which limits the movement of the spacer 14 to that of making a radial rotating movement. The delimiting ring 18 is also provided with a groove 18b extending in the axial direction which enables pressing the spacer 14 into a predetermined position and thus actuation of the assembly 10.

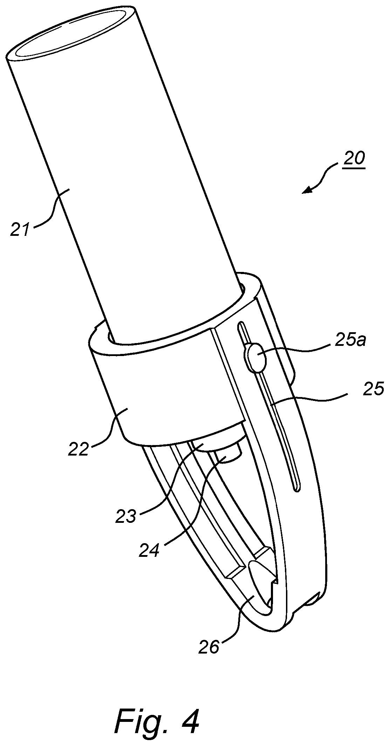

Figure 4 shows an embodiment of the assembly 20 according to the present invention comprising a container 21 and a spacer 22. Also shown in figure 4 is the actuating element 23 which is connected to the nozzle 24. The assembly 20 also shows an adjustable strip 25 having an adjusting knob 25a for moving the adjustable strip 25. The cover means 26 is integrated in the spacer 22.

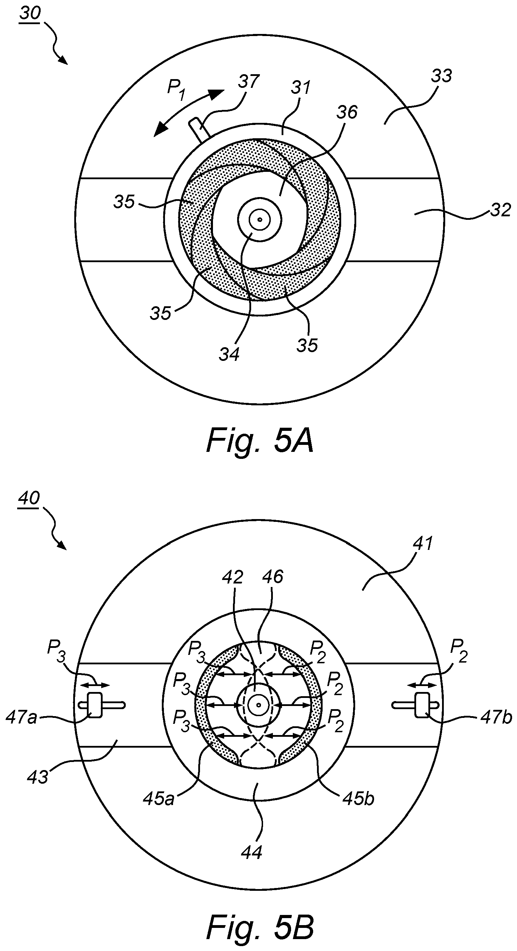

Figure 5a illustrates an embodiment of the assembly 30 comprising a spacer 32 connected to a container 33. The spacer comprises a cover means 31. The container 33 also comprises a nozzle 34 connected to a valve (not visible here). The cover means 31 comprises screen parts 35 that overlap one another in all positions. The screen parts 35 as illustrated in figure 5a together form a diaphragm which describes an opening 36 variable in size. The user can vary the size of opening 36 by moving lever 37 in one of the directions indicated by arrow P₁.

Figure 5b illustrates an embodiment of the assembly 40 comprising a container 41 and a nozzle 42 attached to a valve (not visible here). The container 41 is connected to a spacer 43. The spacer 43 is connected to a cover means 44 comprising screen parts 45a, 45b. The screen parts 45a, 45b in this embodiment are formed by adjustable strips which are separate from one another and which can overlap one another in the opening 46 made in the cover means. The ends of the adjustable strips 45a, 45b preferably describe a V-shape (not illustrated here) or half circle. In figure 5b the ends of the adjustable strips 45a, 45b are located some distance apart, but preferably the ends of the adjustable strips 45a, 45b overlap to prevent the ends of the adjustable strips 45a, 45b from abutting against one another when they are moved together and thus blocking further movement of the adjustable strips 45a, 45b. However, the ends of the adjustable strips 45a, 45b can be shaped such that the adjustable strips 45a, 45b overlap when they are being moved toward one another but without coming to rest against one another.

The spacer 43 comprises at least two adjusting knobs 47a, 47b for manually moving the adjustable strips 45a, 45b. A first adjustable strip 45a can be moved by means of a first adjusting knob 47a by moving the adjusting knob 47a in one of the directions indicated by arrow P₃. As a result of the movement of the adjusting knob 47a, the end of adjustable strip 45a will also move in a direction indicated by arrows P₃. A second adjustable strip 45b, independently of the first adjustable strip 45a, can be moved by means of a second adjusting knob 47b by moving adjusting knob 47b in one of the directions indicated by arrow P₂. As a result of the movement of the adjusting knob 47b the end of adjustable strip 45b will also move in a direction indicated by arrows P₂. The size and shape of opening 46 can be varied by moving one or both ends of adjustable strips 45a, 45b.

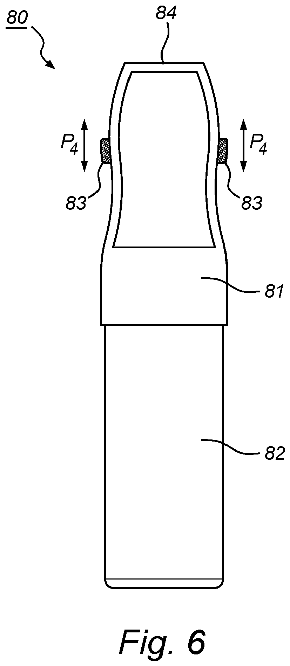

Figure 6 illustrates an embodiment of the assembly 80 comprising a spacer 81 which is connected to a nebulizer 82. The spacer 81 is provided with adjusting knobs 83 for adjustment of the distance between the nozzle (not illustrated here) and the opening 84 provided in the spacer 81 or the surface to be treated (not illustrated here) in the direction of arrows P₄.

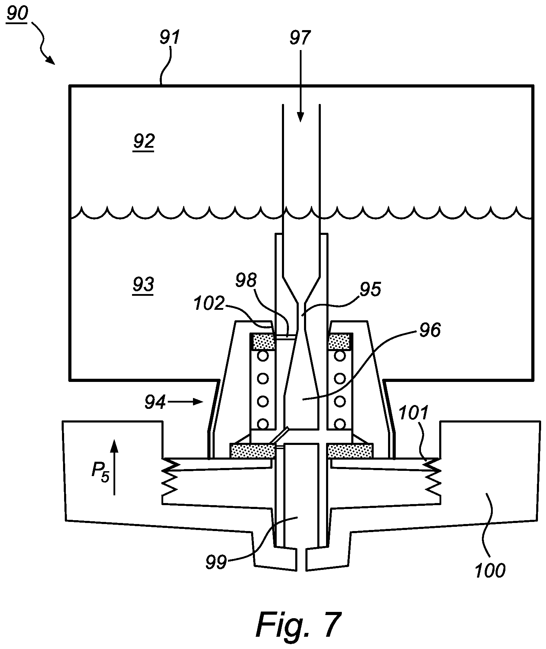

Figure 7 illustrates an embodiment of a nebulizer 90 comprising a container 91 comprising a propellant gas 92 and a liquid 93. The nebulizer 90 also comprises a valve 94 in which a Venturi 95 is affixed which connects with a mixing chamber 96. The valve 94 comprises a first inlet 97 for delivering the propellant gas 92 and a second inlet 98 for delivering the liquid. The valve 94 also comprises a nozzle 99 which is coupled with an actuator 100. The actuator 100 is connected by means of a screw connection 101 to the nozzle 99. Screwing the actuator 100 farther onto the nozzle 99, i.e., in the direction of arrow P₅ means that the nozzle 100 and the valve 94 cannot be pressed in as far. As a result, the second inlet 98 will also be moved over a short distance. As is apparent from the assembly shown, in this way the opening of the second inlet 98 is decreased. The sloping walls 102 affixed in the valve ultimately determine the final bore of the second inlet 98 and therefore the quantity of liquid 93 that is drawn in through the valve 94. Naturally, screwing the actuator 100 farther out in a direction opposite to the direction of arrow P₅ will result in a larger quantity of liquid 93 being able to be drawn up. Then the opening of the second inlet 98 will ultimately come to be completely free with regard to the sloping wall 102 affixed in the valve 94.

## Claims

1. A nebulizer device (1, 10, 20, 30, 40, 50, 80, 90), such as a spray can, for the topical application of a liquid cooling medium, comprising:
- a container (2, 11, 21, 33, 41, 56, 91) comprising a propellant gas (57, 92) and a liquid cooling medium (58, 93), which liquid cooling medium cools the skin by evaporation to a temperature below the freezing point when applied to the skin;
- an actuatable valve (12, 54, 94) attached to the container; and
- a nozzle (3, 13, 24, 34, 42, 55, 99) attached to the valve, designed for generating a stream or spray,
- a spacer (4),
- a cover means (6) with an opening (6a),
wherein the valve:
- comprises a mixing chamber (53, 96) attached to the nozzle; and
- comprises a first (51, 97) and a second inlet (52, 98) attached to the mixing chamber for the separate delivery of the liquid cooling medium and the propellant gas to the mixing chamber,
wherein the mixing chamber is provided with a Venturi (59, 95) and is designed for forming a colloidal mixture comprising the liquid colling medium and the propellant gas before delivering the colloidal mixture to the nozzle,
wherein the device is configured such that when the nozzle is pointed downwards at a lower level than the container:
- the first inlet delivers the propellant gas to the mixing chamber and the second inlet delivers the liquid cooling medium to the mixing chamber;
- upon activating the nebulizer, propellant gas is delivered to the mixing chamber via the first inlet to which the liquid cooling medium is drawn up by the Venturi effect via second inlet;
- the colloidal mixture comprises a dispersed phase consisting of the liquid cooling medium and a continuous phase consisting of the propellant gas; and
wherein the nebulizer is connected to the spacer, which spacer is connected to the cover means provided with the opening, wherein the center axis of the stream or spray to be generated by the nebulizer extends through the opening.

2. The device (1, 10, 20, 30, 40, 50, 80, 90) as claimed in claim 1, **characterized in that** the device comprises at least a first and a second partial container (2, 11, 21, 33, 41, 56, 91), which first partial container holds the propellant gas (57, 92) and is connected to the first inlet and wherein the second partial container holds the liquid cooling medium (58, 93) and is connected to the second inlet.

3. The device (1, 10, 20, 30, 40, 50, 80, 90) as claimed in claim 1 or 2, **characterized in that** the mixing chamber (53, 96) is provided with inlet blocking means that is opened when the first (51, 97) and second (52, 98) inlets are positioned such that they supply the propellant gas (57, 92) to the first inlet and the liquid cooling medium (58, 93) to the second inlet in order to form the colloidal mixture.

4. The device (1, 10, 20, 30, 40, 50, 80, 90) as claimed in any one of the preceding claims, **characterized in that** the stream generated by the nozzle (3, 13, 24, 34, 42, 55, 99) has the shape of a cone and that the top angle of the cone is smaller than 30°, preferably smaller than 20° and preferably smaller than 10°, preferably smaller than 5°.

5. The device (1, 10, 20, 30, 40, 50, 80, 90) as claimed in any one of the preceding claims, **characterized in that** the liquid cooling medium (58, 93) comprises a cooling agent selected from propane, n-butane, 1,1,1,2-tetrafluoroethane, dimethyl ether, dimethyl ether/propane mixture and combinations thereof.

6. The device (1, 10, 20, 30, 40, 50, 80, 90) as claimed in any one of the preceding claims, **characterized in that** the colloidal mixture comprises at least one active compound

7. The device (1, 10, 20, 30, 40, 50, 80, 90) as claimed in any one of the preceding claims, **characterized in that** the propellant gas (57, 92) comprises a mixture of hydrocarbons.

8. The device (1, 10, 20, 30, 40, 50, 80, 90) as claimed in any one of the preceding claims, **characterized in that** the propellant gas (57, 92) comprises a cooling agent, in particular propane, n-butane, 1,1,1,2-tetrafluoroethane, dimethyl ether, dimethyl ether/propane mixture and combinations thereof.

9. The device (1, 10, 20, 30, 40, 50, 80, 90) as claimed in any one of the preceding claims, **characterized in that** the spacer:
- is designed for adjustment of the distance between the nozzle of the nebulizer and the surface to be treated; and
- is designed for fixing the minimal distance between the nozzle attached to the nebulizer and the opening provided in the cover means,
wherein the size of the opening provided in the cover means is variable.

10. The device (1, 10, 20, 30, 40, 50, 80, 90) as claimed in claim 9, **characterized in that** the cover means comprises at least two screen parts movable relative to one another into at least two different positions, and that in the different positions openings with different dimensions are enclosed.

11. The device (1, 10, 20, 30, 40, 50, 80, 90) as claimed in claim 9 or 10, **characterized in that** the cover means comprises at least one screen element adjustable strip provided with openings of different dimensions, which can be moved by guide means belonging to the cover means and/or spacer.

12. Use of a nebulizer device (1, 10, 20, 30, 40, 50, 80, 90) for topical application of a liquid cooling medium to the skin of a person or animal, which liquid cooling medium cools the skin by evaporation to a temperature below the freezing point when applied to the skin, said nebulizer device comprising:
- a container (2, 11, 21, 33, 41, 56, 91) comprising a propellant gas (57, 92) and a liquid cooling medium (58, 93);
- an actuatable valve (12, 54, 94) attached to the container; and
- a nozzle (3, 13, 24, 34, 42, 55, 99) attached to the valve, designed for generating a stream or spray,
wherein the valve:
- comprises a mixing chamber (53, 96) attached to the nozzle; and
- comprises a first (51, 97) and a second inlet (52, 98) attached to the mixing chamber for the separate delivery of the liquid cooling medium and the propellant gas to the mixing chamber,
wherein the mixing chamber is provided with a Venturi (59, 95) and is designed for forming a colloidal mixture comprising the liquid cooling medium and the propellant gas before delivering the colloidal mixture to the nozzle,
wherein the device is configured such that when the nozzle is pointed downwards at a lower level than the container:
- the first inlet delivers the propellant gas to the mixing chamber and the second inlet delivers the liquid cooling medium to the mixing chamber;
- upon activating the nebulizer, propellant gas is delivered to the mixing chamber via the first inlet to which the liquid cooling medium is drawn up by the Venturi effect via second inlet; and
- the colloidal mixture comprises a dispersed phase consisting of the liquid cooling medium and a continuous phase consisting of the propellant gas; and
wherein the use comprises actuating the valve of the device when the nozzle is at a lower level than the container.

13. The use as claimed in claim 12 for the application of cosmetics to a body surface such as the skin.

14. The use as claimed in claim 12 for non-therapeutic treatment of warts, pigmented spots or moles.

## Patentansprüche

1. Eine Zerstäubervorrichtung (1, 10, 20, 30, 40, 50, 80, 90), zum Beispiel eine Sprühdose, für das topische Aufbringen eines flüssigen Kühlmediums, aufweisend:
- einen Behälter (2, 11, 21, 33, 41, 56, 91) aufweisend ein Treibgas (57, 92) und ein flüssiges Kühlmedium (58, 93), welches flüssige Kühlmedium die Haut mittels Verdunstung zu einer Temperatur unter dem Gefrierpunkt kühlt, wenn es auf die Haut aufgebracht wird;
- ein betätigbares Ventil (12, 54, 94), welches an dem Behälter befestigt ist; und
- eine Düse (3, 13, 24, 34, 42, 55, 99), welche an dem Ventil befestigt ist, welche zum Erzeugen eines Strahls oder Sprühnebels eingerichtet ist,
- einen Abstandshalter (4),
- ein Abdeckungsmittel (6) mit einer Öffnung (6a),
wobei das Ventil:
- eine Mischkammer (53, 96) aufweist, welche an der Düse befestigt ist; und
- einen ersten (51, 97) und einen zweiten Einlass (52, 98) aufweist, welche an der Mischkammer befestigt sind, für die getrennte Zufuhr des flüssigen Kühlmediums und des Treibgases zu der Mischkammer,
wobei die Mischkammer mit einem Venturi (59, 95) bereitgestellt ist, und zum Bilden einer kolloidalen Mischung eingerichtet ist, welche das flüssige Kühlmedium und das Treibgas aufweist, bevor die kolloidale Mischung der Düse zugeführt wird,
wobei die Vorrichtung so konfiguriert ist, dass, wenn die Düse nach unten zeigt bei einer niedrigeren Höhe als der Behälter:
- der erste Einlass das Treibgas der Mischkammer zuführt, und der zweite Einlass das flüssige Kühlmedium der Mischkammer zuführt;
- auf ein Aktivieren des Zerstäubers hin das Treibgas via den ersten Einlass der Mischkammer zugeführt wird, zu welchem Einlass das flüssige Kühlmedium via den zweiten Einlass mittels des Venturi-Effekts gezogen wird;
- die kolloidale Mischung eine dispergierte Phase aufweist, welche aus dem flüssigen Kühlmedium besteht, und eine kontinuierliche Phase aufweist, welche aus dem Treibgas besteht; und
wobei der Zerstäuber mit dem Abstandshalter verbunden ist, welcher Abstandshalter mit dem Abdeckungsmittel verbunden ist, welches mit der Öffnung bereitgestellt ist, wobei sich die Mittelachse des Strahls oder Sprühnebels, welcher mittels des Zerstäubers zu erzeugen ist, durch die Öffnung erstreckt.

2. Die Vorrichtung (1, 10, 20, 30, 40, 50, 80, 90) gemäß Anspruch 1, **dadurch gekennzeichnet, dass** die Vorrichtung mindestens einen ersten und einen zweiten Teilbehälter (2, 11, 21, 33, 41, 56, 91) aufweist, welcher erste Teilbehälter das Treibgas (57, 92) enthält und mit dem ersten Einlass verbunden ist, und wobei der zweite Teilcontainer das flüssige Kühlmedium (58, 93) enthält und mit dem zweiten Einlass verbunden ist.

3. Die Vorrichtung (1, 10, 20, 30, 40, 50, 80, 90) gemäß Anspruch 1 oder 2, **dadurch gekennzeichnet, dass** die Mischkammer (53, 96) mit einem Einlassblockiermittel bereitgestellt ist, welches geöffnet wird, wenn der erste (51, 97) und der zweite (52, 98) Einlass so positioniert sind, dass sie das Treibgas (57, 92) dem ersten Einlass zuführen und das flüssige Kühlmedium (58, 93) dem zweiten Einlass zuführen, um die kolloidale Mischung zu bilden.

4. Die Vorrichtung (1, 10, 20, 30, 40, 50, 80, 90) gemäß irgendeinem der vorangehenden Ansprüche, **dadurch gekennzeichnet, dass** der Strahl, welcher mittels der Düse (3, 13, 24, 34, 42, 55, 99) erzeugt wird, die Form eines Kegels hat, und dass der obere Winkel des Kegels kleiner als 30° ist, bevorzugt kleiner als 20°, und bevorzugt kleiner als 10°, vorzugsweise kleiner als 5°.

5. Die Vorrichtung (1, 10, 20, 30, 40, 50, 80, 90) gemäß irgendeinem der vorangehenden Ansprüche, **dadurch gekennzeichnet, dass** das flüssige Kühlmedium (58, 93) ein Kühlmittel aufweist, welches ausgewählt ist aus Propan, n-Butan, 1,1,1,2-Tetrafluorethan, Dimethylether, einer Dimethylether/Propan Mischung, und Kombinationen daraus.

6. Die Vorrichtung (1, 10, 20, 30, 40, 50, 80, 90) gemäß irgendeinem der vorangehenden Ansprüche, **dadurch gekennzeichnet, dass** die kolloidale Mischung mindestens eine aktive Verbindung aufweist.

7. Die Vorrichtung (1, 10, 20, 30, 40, 50, 80, 90) gemäß irgendeinem der vorangehenden Ansprüche, **dadurch gekennzeichnet, dass** das Treibgas (57, 92) eine Mischung aus Kohlenwasserstoffen aufweist.

8. Die Vorrichtung (1, 10, 20, 30, 40, 50, 80, 90) gemäß irgendeinem der vorangehenden Ansprüche, **dadurch gekennzeichnet, dass** das Treibgas (57, 92) ein Kühlmittel aufweist, insbesondere Propan, n-Butan, 1,1,1,2-Tetrafluorethan, Dimethylether, eine Dimethylether/Propan Mischung, und Kombinationen daraus.

9. Die Vorrichtung (1, 10, 20, 30, 40, 50, 80, 90) gemäß irgendeinem der vorangehenden Ansprüche, **dadurch gekennzeichnet, dass** der Abstandshalter:
- für eine Einstellung des Abstands zwischen der Düse des Zerstäubers und der zu behandelnden Oberfläche eingerichtet ist; und
- zum Fixieren des minimalen Abstands zwischen der Düse, welche an dem Zerstäuber befestigt ist, und der Öffnung eingerichtet ist, welche in dem Abdeckungsmittel bereitgestellt ist,
wobei die Größe der Öffnung, welche in dem Abdeckungsmittel bereitgestellt ist, variabel ist.

10. Die Vorrichtung (1, 10, 20, 30, 40, 50, 80, 90) gemäß Anspruch 9, **dadurch gekennzeichnet, dass** das Abdeckungsmittel mindestens zwei Abschirmungsteile aufweist, welche relativ zueinander zu mindestens zwei verschiedenen Positionen beweglich sind, und dass in den verschiedenen Positionen Öffnungen mit verschiedenen Dimensionen umschlossen sind.

11. Die Vorrichtung (1, 10, 20, 30, 40, 50, 80, 90) gemäß Anspruch 9 oder 10, **dadurch gekennzeichnet, dass** das Abdeckungsmittel mindestens einen vom Abschirmungselement einstellbaren Streifen aufweist, welcher mit Öffnungen mit verschiedenen Dimensionen bereitgestellt ist, welcher mittels eines Führungsmittels bewegt werden kann, welches zu dem Abdeckungsmittel und/oder Abstandshalter gehört.

12. Verwendung einer Zerstäubervorrichtung (1, 10, 20, 30, 40, 50, 80, 90) für ein topisches Aufbringen eines flüssigen Kühlmediums auf die Haut einer Person oder eines Tieres, welches flüssiges Kühlmedium die Haut mittels Verdunstung zu einer Temperatur unter dem Gefrierpunkt kühlt, wenn sie auf die Haut aufgebracht wird, wobei die Zerstäubervorrichtung aufweist:
- einen Behälter (2, 11, 21, 33, 41, 56, 91) aufweisend ein Treibgas (57, 92) und ein flüssiges Kühlmedium (58, 93);
- ein betätigbares Ventil (12, 54, 94), welches an dem Behälter befestigt ist; und
- eine Düse (3, 13, 24, 34, 42, 55, 99), welche an dem Ventil befestigt ist, welche zum Erzeugen eines Strahls oder Sprühnebels eingerichtet ist,
wobei das Ventil:
- eine Mischkammer (53, 96) aufweist, welche an der Düse befestigt ist; und
- einen ersten (51, 97) und einen zweiten Einlass (52, 98) aufweist, welche an der Mischkammer befestigt sind, für die getrennte Zufuhr des flüssigen Kühlmediums und des Treibgases zu der Mischkammer,
wobei die Mischkammer mit einem Venturi (59, 95) bereitgestellt ist, und zum Bilden einer kolloidalen Mischung eingerichtet ist, welche das flüssige Kühlmedium und das Treibgas aufweist, bevor die kolloidale Mischung der Düse zugeführt wird,
wobei die Vorrichtung so konfiguriert ist, dass, wenn die Düse nach unten zeigt bei einer niedrigeren Höhe als der Behälter:
- der erste Einlass das Treibgas der Mischkammer zuführt, und der zweite Einlass das flüssige Kühlmedium der Mischkammer zuführt;
- auf ein Aktivieren des Zerstäubers hin das Treibgas via den ersten Einlass der Mischkammer zugeführt wird, zu welchem Einlass das flüssige Kühlmedium via den zweiten Einlass mittels des Venturi-Effekts gezogen wird;
- die kolloidale Mischung eine dispergierte Phase aufweist, welche aus dem flüssigen Kühlmedium besteht, und eine kontinuierliche Phase aufweist, welche aus dem Treibgas besteht; und
wobei die Verwendung das Betätigen des Ventils der Vorrichtung aufweist, wenn die Düse bei einer niedrigeren Höhe als der Behälter ist.

13. Die Verwendung gemäß Anspruch 12 für das Aufbringen von Kosmetik auf eine Körperoberfläche, zum Beispiel die Haut.

14. Die Verwendung gemäß Anspruch 12 für eine nicht-therapeutische Behandlung von Warzen, Pigmentflecken, oder Muttermalen.

## Revendications

1. - Dispositif nébuliseur (1, 10, 20, 30, 40, 50, 80, 90), tel qu'une bombe aérosol, pour l'application topique d'un milieu de refroidissement liquide, comprenant :
- un récipient (2, 11, 21, 33, 41, 56, 91) comprenant un gaz propulseur (57, 92) et un milieu de refroidissement liquide (58, 93), lequel milieu de refroidissement liquide refroidit la peau par évaporation à une température au-dessous du point de congélation lorsqu'il est appliqué sur la peau ;
- une soupape actionnable (12, 54, 94) attachée au récipient ; et
- une buse (3, 13, 24, 34, 42, 55, 99) attachée à la soupape, conçue pour générer un courant ou une pulvérisation ;
- un espaceur (4) ;
- un moyen couvercle (6) avec une ouverture (6a) ;
dans lequel la soupape :
- comprend une chambre de mélange (53, 96) attachée à la buse ; et
- comprend une première (51, 97) et une seconde entrée (52, 98) attachées à la chambre de mélange pour la distribution séparée du milieu de refroidissement liquide et du gaz propulseur à la chambre de mélange,
dans lequel la chambre de mélange est dotée d'un Venturi (59, 95) et est conçue pour former un mélange colloïdal comprenant le milieu de refroidissement liquide et le gaz propulseur avant de distribuer le mélange colloïdal à la buse,
dans lequel le dispositif est configuré de telle sorte que, lorsque la buse est dirigée vers le bas à un niveau inférieur au récipient :
- la première entrée distribue le gaz propulseur à la chambre de mélange et la seconde entrée distribue le milieu de refroidissement liquide à la chambre de mélange ;
- lors de l'activation du nébuliseur, du gaz propulseur est distribué à la chambre de mélange par l'intermédiaire de la première entrée à laquelle le milieu de refroidissement liquide est aspiré par l'effet Venturi par l'intermédiaire de la seconde entrée ;
- le mélange colloïdal comprend une phase dispersée consistant en le milieu de refroidissement liquide et une phase continue consistant en le gaz propulseur ; et
dans lequel le nébuliseur est relié à l'espaceur, lequel espaceur est relié au moyen couvercle doté de l'ouverture, l'axe central du courant ou de la pulvérisation à générer par le nébuliseur s'étendant à travers l'ouverture.

2. - Dispositif (1, 10, 20, 30, 40, 50, 80, 90) selon la revendication 1, **caractérisé par le fait que** le dispositif comprend au moins un premier et un second récipient partiel (2, 11, 21, 33, 41, 56, 91), lequel premier récipient partiel contient le gaz propulseur (57, 92) et est relié à la première entrée, et dans lequel le second récipient partiel contient le milieu de refroidissement liquide (58, 93) et est relié à la seconde entrée.

3. - Dispositif (1, 10, 20, 30, 40, 50, 80, 90) selon l'une des revendications 1 ou 2, **caractérisé par le fait que** la chambre de mélange (53, 96) est doté d'un moyen de blocage d'entrée qui est ouvert lorsque les première (51, 97) et seconde (52, 98) entrées sont positionnées de telle sorte qu'elles fournissent le gaz propulseur (57, 92) à la première entrée et le milieu de refroidissement liquide (58, 93) à la seconde entrée de façon à former le mélange colloïdal.

4. - Dispositif (1, 10, 20, 30, 40, 50, 80, 90) selon l'une quelconque des revendications précédentes, **caractérisé par le fait que** le courant généré par la buse (3, 13, 24, 34, 42, 55, 99) a la forme d'un cône, et que l'angle supérieur du cône est inférieur à 30°, de préférence inférieur à 20° et de préférence inférieur à 10°, de préférence inférieur à 5°.

5. - Dispositif (1, 10, 20, 30, 40, 50, 80, 90) selon l'une quelconque des revendications précédentes, **caractérisé par le fait que** le milieu de refroidissement liquide (58, 93) comprend un agent de refroidissement choisi parmi le propane, le n-butane, le 1,1,1,2-tétrafluoroéthane, l'éther diméthylique, un mélange éther diméthylique/propane et les combinaisons de ceux-ci.

6. - Dispositif (1, 10, 20, 30, 40, 50, 80, 90) selon l'une quelconque des revendications précédentes, **caractérisé par le fait que** le mélange colloïdal comprend au moins un composé actif.

7. - Dispositif (1, 10, 20, 30, 40, 50, 80, 90) selon l'une quelconque des revendications précédentes, **caractérisé par le fait que** le gaz propulseur (57, 92) comprend un mélange d'hydrocarbures.

8. - Dispositif (1, 10, 20, 30, 40, 50, 80, 90) selon l'une quelconque des revendications précédentes, **caractérisé par le fait que** le gaz propulseur (57, 92) comprend un agent de refroidissement, en particulier le propane, le n-butane, le 1,1,1,2-tétrafluoroéthane, l'éther diméthylique, un mélange diméthylique/propane et les combinaisons de ceux-ci.

9. - Dispositif (1, 10, 20, 30, 40, 50, 80, 90) selon l'une quelconque des revendications précédentes, **caractérisé par le fait que** l'espaceur :
- est conçu pour l'ajustement de la distance entre la buse du nébuliseur et la surface à traiter ; et
- est conçu pour fixer la distance minimale entre la buse attachée au nébuliseur et l'ouverture disposée dans le moyen couvercle,
dans lequel la dimension de l'ouverture disposée dans le moyen couvercle est variable.

10. - Dispositif (1, 10, 20, 30, 40, 50, 80, 90) selon la revendication 9, **caractérisé par le fait que** le moyen couvercle comprend au moins deux parties écrans déplaçables l'une par rapport à l'autre dans au moins deux positions différentes, et **par le fait que**, dans les différentes positions, des ouvertures ayant des dimensions différentes sont enfermées.

11. - Dispositif (1, 10, 20, 30, 40, 50, 80, 90) selon l'une des revendications 9 ou 10, **caractérisé par le fait que** le moyen couvercle comprend au moins une bande réglable d'élément écran dotée d'ouvertures de différentes dimensions, qui peut être déplacée par des moyens de guidage appartenant au moyen couvercle et/ou à l'espaceur.

12. - Utilisation d'un dispositif nébuliseur (1, 10, 20, 30, 40, 50, 80, 90) pour l'application topique d'un milieu de refroidissement liquide sur la peau d'une personne ou d'un animal, lequel milieu de refroidissement liquide refroidit la peau par évaporation à une température au-dessous du point de congélation lorsqu'il est appliqué sur la peau, ledit dispositif nébuliseur comprenant :
- un récipient (2, 11, 21, 33, 41, 56, 91) comprenant un gaz propulseur (57, 92) et un milieu de refroidissement liquide (58, 93) ;
- une soupape actionnable (12, 54, 94) attachée au récipient ; et
- une buse (3, 13, 24, 34, 42, 55, 99) attachée à la soupape, conçue pour générer un courant ou une pulvérisation ;
dans lequel la soupape :
- comprend une chambre de mélange (53, 96) attachée à la buse ; et
- comprend une première (51, 97) et une seconde entrée (52, 98) fixées à la chambre de mélange pour la distribution séparée du milieu de refroidissement liquide et du gaz propulseur à la chambre de mélange,
dans lequel la chambre de mélange est dotée d'un Venturi (59, 95) et est conçue pour former un mélange colloïdal comprenant le milieu de refroidissement liquide et le gaz propulseur avant de distribuer le mélange colloïdal à la buse,
dans lequel le dispositif est configuré de telle sorte que, lorsque la buse est dirigée vers le bas à un niveau inférieur au récipient :
- la première entrée distribue le gaz propulseur à la chambre de mélange et la seconde entrée distribue le milieu de refroidissement liquide à la chambre de mélange ;
- lors de l'activation du nébuliseur, du gaz propulseur est distribué à la chambre de mélange par l'intermédiaire de la première entrée à laquelle le milieu de refroidissement liquide est aspiré par l'effet Venturi par l'intermédiaire de la seconde entrée ; et
- le mélange colloïdal comprend une phase dispersée consistant en le milieu de refroidissement liquide et une phase continue consistant en le gaz propulseur ; et
dans lequel l'utilisation comprend l'actionnement de la soupape du dispositif lorsque la buse est à un niveau inférieur au récipient.

13. - Utilisation selon la revendication 12 pour l'application de produits cosmétiques sur une surface corporelle, telle que la peau.

14. - Utilisation selon la revendication 12 pour le traitement non-thérapeutique de verrues, de taches pigmentées ou de grains de beauté.
